# EUROPEAN PATENT SPECIFICATION

(11) **EP 4 507 762 B1**
(45) Date of publication and mention of the grant of the patent: **18.03.2026**
(21) Application number: 23714585.9
(22) Date of filing: 05.04.2023
(51) Int. Cl.: A61M 5/32

(54) **CAP FOR REMOVING A NEEDLE SHIELD FROM A MEDICAL CONTAINER, AUTOINJECTOR INCLUDING SAID CAP, AND METHOD FOR ASSEMBLING SAID MEDICAL CONTAINER AND SAID AUTOINJECTOR**
KAPPE ZUM ENTFERNEN EINES NADELSCHUTZES VON EINEM MEDIZINISCHEN BEHÄLTER, AUTOINJEKTOR MIT DIESER KAPPE UND VERFAHREN ZUR MONTAGE DES MEDIZINISCHEN BEHÄLTERS UND DES AUTOINJEKTORS
CAPUCHON POUR RETIRER UN PROTÈGE-AIGUILLE D'UN RÉCIPIENT MÉDICAL, AUTO-INJECTEUR COMPRENANT LEDIT CAPUCHON ET PROCÉDÉ D'ASSEMBLAGE DUDIT RÉCIPIENT MÉDICAL ET DUDIT AUTO-INJECTEUR

(30) Priority: 11.04.2022 EP 22305520
(43) Date of publication of application: 19.02.2025
(73) Proprietor: Becton Dickinson France, 38800 Le Pont-de-Claix (FR)
(72) Inventor: CARREL, Franck, 38220 Saint Jean de Vaulx (FR)
(74) Representative: Germain Maureau
(86) International application number: PCT/EP2023/059047
(87) International publication number: WO 2023/198567

(56) References cited:
- WO-A1-2005/079889
- GB-A- 2 465 389

## Description

The present invention relates to a cap for removing a needle shield from a medical container, an autoinjector provided with this cap, and a method for assembling a medical container and the autoinjector.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Automatic injection devices are designed for automatic injection of a medical product into an injection site. Autoinjectors usually comprise a housing for receiving a medical container. The medical container has a barrel defining a reservoir for containing the medical product, the barrel having a distal end provided with an injection needle and an opened proximal end receiving a plunger rod for pushing a stopper. The opened proximal end is usually provided with a flange.

Autoinjectors also include a safety shield mechanism moving from an extended to a retracted position to respectively shield or unveil the needle and an injection mechanism for automatically injecting the medical into an injection site. The injection mechanism usually includes a plunger rod for pushing a stopper inside the barrel of the medical container, and an initially compressed spring for moving the plunger in the distal direction. Locking means are provided for maintaining the plunger rod in an initial position in which the plunger rod is axially blocked despite the action of the compressed spring. A release member is typically arranged to release the plunger from the locking means and allow the spring to push the plunger rod in the distal direction to perform injection. A predetermined displacement of the safety shield towards the retracted position is required to allow the release member to unlock the locking means and release the plunger rod.

The document EP2921191 discloses a device for automatic injection of a medical product into an injection site. The device includes a two-part assembly having an upper sub-assembly assembled to a lower sub-assembly. The lower sub-assembly receives a syringe provided with an injection needle. The injection needle is covered by a rigid needle shield. The device further includes a needle cover movable relative to the housing between an extended and a retracted position. The plunger rod is coupled to a compressed spring and maintained in an initial position by flexible teeth of an inner cylinder. A push button is arranged at a proximal end of the device for releasing the plunger rod by deflecting the flexible teeth outwardly. Activation of the push button only becomes possible when the needle cover has moved a predetermined distance in the proximal direction towards the retracted position. The device also includes a cap provided with clamping means for abutting against a proximal end of the rigid needle shield such that removal of the cap entails removal of the rigid needle shield. The cap includes a central hole for accommodating the rigid needle shield during assembly.

The syringe is distally inserted into the lower sub-assembly. During this insertion, the clamping means of the cap are deflected by the syringe barrel. If nothing is done, the clamping means may tend to creep over time, i.e. stay deflected, the risk being that later, the clamping means fail to properly engage the needle shield during removal of the cap. There is therefore a need for a "push back" step in which the rigid needle shield is pushed back in the proximal direction so that the clamping means may return to a rest position in which the clamping means can reliable engage the needle shield. This push-back step may be operated by a machine engaging the central hole of the cap for pushing the needle shield back in the proximal direction. However, this operation is time-consuming. Besides, the central hole of the cap makes the needle shield visible to the end user. This may be confusing for the end user who is generally not a skilled healthcare worker.

The document GB2465389 discloses a syringe needle cover remover. The document WO2005079889 discloses a handling device for use with a medical injector including a protective sheath.

There is therefore a need for a cap that enables to avoid the above-mentioned creep effect and that permits a more efficient push-back step during assembly of the medical container within the autoinjector.

The invention is defined in independent claims 1 and 15. Advantageous embodiments are subject of the dependent claims.

An aspect of the invention is a cap for removing a needle shield attached to a medical container provided with an injection needle, the cap including:
a tubular body extending along a longitudinal axis A, the tubular body having a distal end and an opposite proximal end for attachment to a bottom housing of an autoinjector,
an inner sleeve proximally extending inside the tubular body from said distal end, the inner sleeve defining an inner cavity for receiving the needle shield,
clamping means for clamping the needle shield when the needle shield is inserted inside the inner cavity, the clamping means including an axial abutment surface for abutting against a proximal end of the needle shield,
a plug at least partially extending inside the inner cavity, the plug having a distal end portion, a proximal end portion and an intermediate portion extending between the distal end portion and the proximal end portion,
wherein at least the proximal end portion of the plug is movable between a first position, in which the proximal end portion allows the proximal end of the needle shield to distally pass the axial abutment surface of the clamping means, and a second position, in which the proximal end portion of the plug moves in the proximal direction to push the needle shield back towards the axial abutment surface.

The cap of the invention thus enables to push the needle shield and the medical container back in the proximal direction such that the clamping means may return to their rest position, in which the hooks extend between a distal shoulder of the syringe barrel and the proximal end of the needle shield. As a result, the clamping means do not remain in a deflected state over a long time. Instead, the clamping means stay in their rest position, i.e. undeformed, during the storage period. When the end user pulls the cap in the distal direction, the hooks can thus reliably engage the proximal end of the needle shield. This engagement entails removal of the needle shield.

The cap of the invention may further include some or all of the features below.

In an embodiment, the distal end portion of the plug and a distal opening of the inner cavity have a complementary shape.

In an embodiment, the intermediate portion of the plug includes one or more axial walls for centering and securing the plug inside the inner cavity.

In an embodiment, the proximal end portion of the plug includes a chamfer.

In an embodiment, the distal end portion of the plug is flush with regard to the distal end of the tubular body in the second position.

In an embodiment, the distal end portion of the plug has a proximal shoulder for abutting against an axial shoulder of the inner cavity in the second position.

In an embodiment, a diameter of the proximal end portion is lower than a diameter of the distal end portion.

In an embodiment, the proximal end portion and the distal end portion of the plug are symmetrical with regard to a transversal plane orthogonal to the longitudinal axis A.

The proximal end portion and the distal end portion of the plug may thus be identical.

In an embodiment, the intermediate portion of the plug includes a spring portion for biasing the proximal end portion in the proximal direction.

In an embodiment, the proximal end portion of the plug is disc-shaped.

In an embodiment, the proximal end portion of the plug is formed by a proximal end of said one or more axial walls.

In an embodiment, the plug is made of a single piece.

In an embodiment, the plug is rotationally symmetrical around the longitudinal axis A.

The plug may be axially movable between a pre-assembled position in which the proximal end portion is away from the needle shield and an assembled position in which the proximal end portion abuts against the needle shield. In the pre-assembled position, the plug may partially extend outside the inner cavity and may protrude from the distal end of the tubular body, while in the assembled position, the plug may be fully inserted inside the inner cavity.

Another aspect of the invention is an autoinjector including a lower sub-assembly provided with the above-described cap.

Another aspect of the invention is a method for assembling the above-described autoinjector and a medical container having a needle shield, the method including the steps of:
- inserting the medical container inside the lower sub-assembly of the autoinjector and the needle shield inside the inner cavity of the cap until a proximal end of the needle shield distally passes over the axial abutment surface of the clamping means;
- pushing the needle shield back in the proximal direction towards the axial abutment surface of the clamping means, this movement of the needle shield being caused by the proximal end portion of the plug moving from the first position to the second position.

The invention and the advantages arising therefrom will clearly emerge from the detailed description that is given below with reference to the appended drawings as follows :
- Figure 1A is a perspective cross-section view of an autoinjector lower sub-assembly including a cap according to an embodiment of the invention,
- Figure 1B is a perspective view of a plug for a cap according to an embodiment of the invention,
- Figures 1C and 1D are cross-section views of a cap according to an embodiment of the invention, respectively in pre-assembled position and an assembled position,
- Figure 2A is a perspective view of a plug for a cap according to an embodiment of the invention,
- Figure 2B is a cross-section view of a cap according to an embodiment of the invention, in an assembled position,
- Figure 3A is a perspective view of a plug for a cap according to an embodiment of the invention,
- Figures 3B and 3C are cross-section views of a cap according to an embodiment of the invention, respectively in a first position and in a second position.

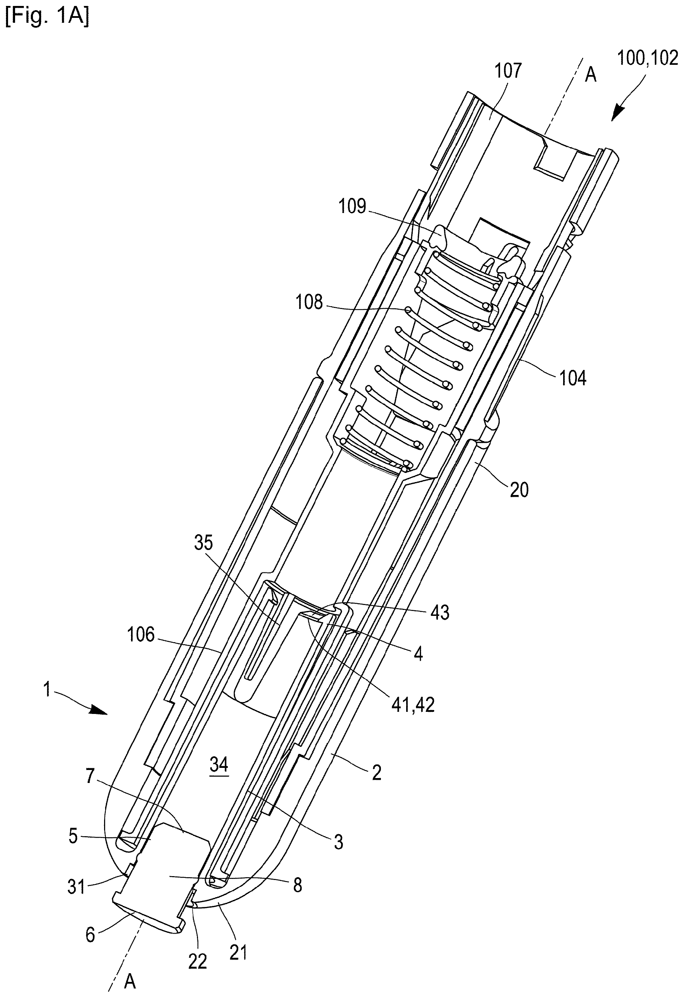

With reference to Figure 1A-1D is shown a cap 1, according to an embodiment of the invention, for removing a needle shield 110 from a medical container 120 having an injection needle 121.

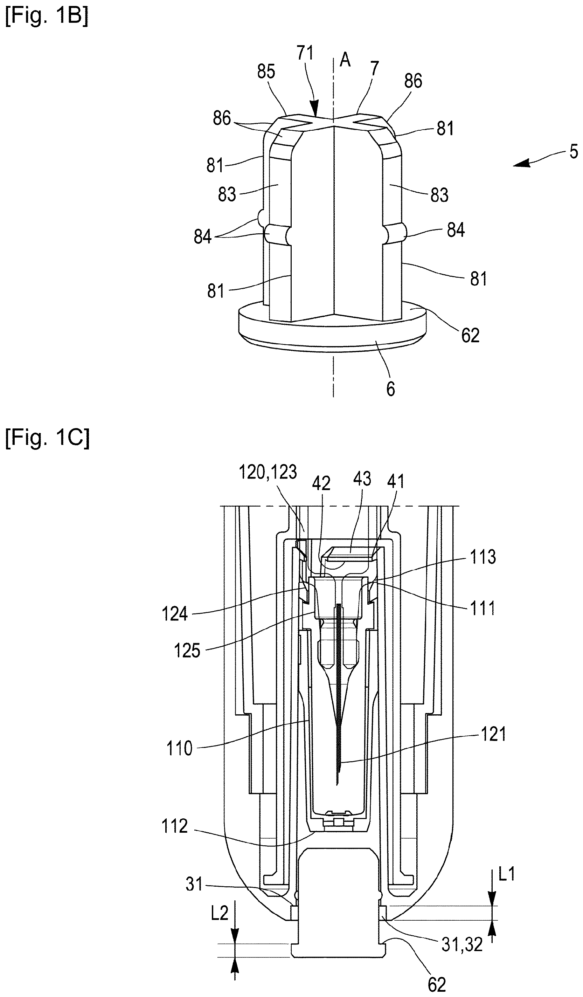

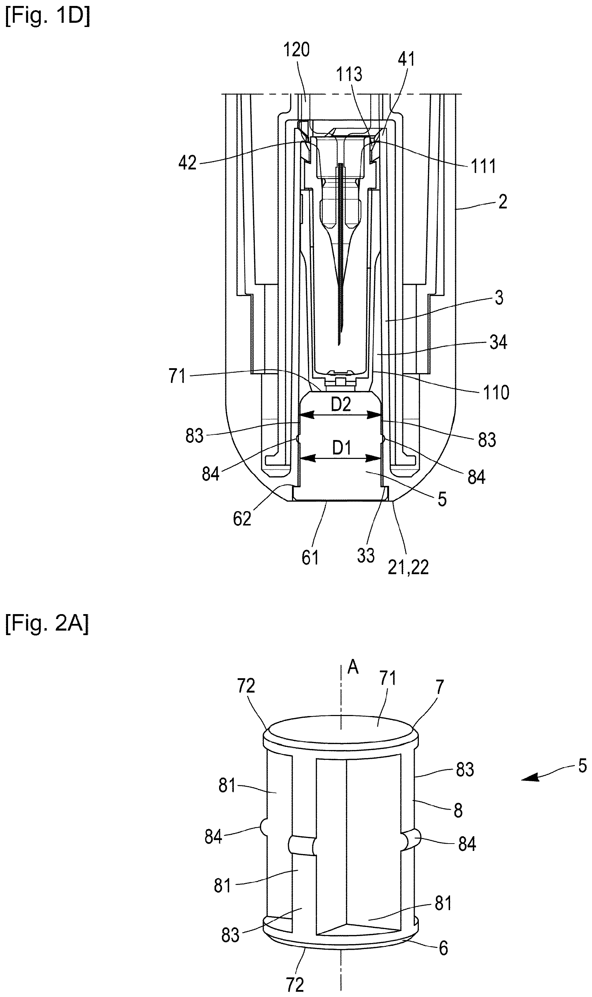

As visible in Figure 1A, the cap 1 is intended to equip a lower sub-assembly 102 of an autoinjector 100 extending along a longitudinal axis A. The lower sub-assembly 102 of the autoinjector 100 is configured to receive a medical container 120 (Figures 1C and 1D), such as a prefilled syringe. As visible in Firgure 1A, the lower sub-assembly 102 may include a bottom housing 104, a needle cover 106, axially movable relative to the lower sub-assembly 102 between an extended position, in which the needle cover 106 is intended to cover the injection needle 121, and a retracted position, in which the needle cover 106 unveils the injection needle 121, a safety spring 108 for moving the needle cover 106 in the distal direction, a ring 109 intended to receive a flange of the medical container 120 so as to move axially together with the medical container 120, and a cam 107 rotationally movable around the longitudinal axis A, movement of the cam 107 being caused by the needle cover 106 moving between the extended and retracted positions.

With reference to Figures 1C and 1D, The medical container 120 has a barrel 123 forming a reservoir for containing a medical product and a longitudinal tip distally protruding from a distal shoulder 124 of the barrel 123. The distal tip 125 defines an axial passageway in fluid communication with the reservoir. The medical container 120 further includes an injection needle 121 inserted in the axial passageway. A needle shield 110 is removably attached to the distal tip 125 of the medical container 120 to seal and protect the needle.

The needle shield 110 has a distal end 112 and an opposite proximal end 111. The proximal end 111 of the needle shield 110 and the distal shoulder 124 of the barrel 123 are configured to face each other and are separated by an axial gap 113.

As visible in Figure 1A, the cap 1 includes a tubular body 2 having a distal end 21 and an opposite proximal end 20 for attachement to the bottom housing 104 of the autoinjector 100. The cap 1 may be attached to the bottom housing 104 by any appropriate means such, for example, friction-fit or snap-fitting means. The distal end 21 may include a circumferential flat surface 22, orthogonal to the longitudinal axis A, which may delimit a distal opening 31.

The cap 1 also includes an inner sleeve 3 axially extending inside the tubular body 2, from the distal end 21 of the cap 1. The tubular body 2 and the inner sleeve 3 may be made of a single piece. The inner sleeve 3 defines an inner cavity 34 configured to receive the needle shield 110. The inner cavity 34 leads to an opened proximal end 35 for allowing the needle shield 110 to penetrate inside the inner sleeve 3. Opposite said opened proximal end 35, the inner cavity 34 leads to the distal opening 31. The inner sleeve 3 has clamping means, such as one or more axially extending fexible legs 4, configured to clamp the needle shield 110. The clamping means are configured such that removal of the cap 1 from the bottom housing 104 entails removal of the needle shield 110 from the medical container 120. The flexible legs 4 may be regularly distributed around the longitudinal axis A. There may be four legs 4 as shown in Figure 1A, although the number of legs 4 may vary and may be less than four or more than four.

The flexible legs 4 have a hook 41 arranged at their proximal end. The flexible legs 4 are radially movable between a rest position, in which the hooks 41 are configured to engage the axial gap 113 separating the barrel 123 and the needle shield 110, and an outwardly deflected position, in which the legs 4 are deflected to allow insertion of the needle shield 110 inside the inner cavity 34. The hooks 41 include an axial abutment surface 42 configured to face and/or abuts against the proximal end 111 of the needle shield 110 and, opposite the axial abutment surface 42, a proximal ramp 43 inclined with respect to the longitudinal axis A for allowing the legs 4 to outwardly deflect when the needle shield 110 is introduced inside the inner sleeve 3.

With reference to Figures 1A and 1B, the cap 1 includes a plug 5 arranged inside the inner cavity 34 for closing the distal opening 31. As a result, the plug 5 hides the needle shield 110 to the end user. With reference to Figures 1B, 2A or 3A, the plug 5 includes a distal end portion 6, an opposite proximal end portion 7 and an intermediate portion 8 axially extending between the distal and the proximal end portions 6, 7.

The plug 5 of Figure 1B is axially movable with regard to the inner sleeve 3 between a pre-assembled position (Figure 1C) in which the plug 5 partially extends outside the inner cavity 34 and distally protrudes from the distal end 21 of the cap 1, and an assembled position (Figure 1D) in which the plug 5 is fully inserted within the inner sleeve 3.

As illustrated in Figure 1B, the distal end portion 6 of the plug 5 is shaped to seal the distal opening 31. To that end, the distal end portion 6 of the plug 5 and the distal opening 31 may be complementarily-shaped, and for instance the distal end portion 6 of the plug 5 may be disc-shaped to fit the distal opening 31. The distal end portion 6 of the plug 5 has a distal surface configured to allow application of a proximal force for pushing the plug 5 in the proximal direction inside the inner cavity 34. This distal surface may be flat and orthogonal to the longitudinal axis A.

With reference to Figure 1C, the distal opening 31 of the cap 1 may include an axial shoulder 33 for stopping insertion of the plug 5 inside the inner cavity 34 at a predetermined location. The axial shoulder 33 may delimit a recess 32 for accommodating the distal end portion 6 of the plug 5, which may be complementarily shaped with said recess 32. More specifically, the axial dimension L1 of the recess 32 may be similar to the axial dimension L2 of the distal end portion 6. The plug 5 thus does not distally protrude from the distal end 21 of the cap 1 when in the assembled position (Figure 1D), i.e. when a proximal shoulder 62 of the distal end portion 6 abuts against the axial shoulder 33 of the distal opening 31. Preferably, the distal suface 61 of the plug 5 is flush with the distal end 21 of the body (and more specifically with the circumferential flat surface 22 of said distal end 21).

The intermediate portion 8 of the plug 5 is configured to center and maintain the plug 5 inside the inner cavity 34. As illustrated in Figure 1B, the intermediate portion 8 of the plug 5 may include one or more axial walls 81 extending parallel to the longitudinal axis A. In the embodiment shown in Figure 1B, the plug 5 comprises four axial walls 81, however the plug 5 may include more or less than four axial walls 81, such as for instance three, five or six axial walls 81. The axial walls 81 may include the central longitudinal axis A and cross each other along said central longitudinal axis A. The axial walls 81 may extend at regular angles from each other around the longitudinal axis A, for instance they may be separated by a 90° angle as shown in Figure 1B.

The plug 5, more specifically the intermediate portion 8 of the plug 5, further includes securing means for securing the plug 5 inside the inner cavity 34. The securing means may include an axial edge 83 of the axial walls 81. Furthermore, they may include one or more outwardly extending radial protrusions 84 configured to abut against a lateral surface of the inner cavity 34 or to engage a recess 32 (not shown) arranged on said lateral surface. To that end, the diameter D1 (see Figure 1D) between two diametrically opposite axial edge 83 or two diametrically opposite protrusions may be higher than an inner diameter D2 of the inner sleeve 3. Preferably, the securing means are thus friction-fit or snap-fitting means. As illustrated in Figure 1B, the radial protrusions 84 may be arranged on the axial edge 83 of the axial walls 81, and for instance at the middle of the axial length of the plug 5.

The axial edges 83 of the axial walls 81 are arranged at a distance from an edge of the distal end portion 6, thereby delimiting the proximal shoulder 62 of the distal end portion 6 of the plug 5.

Still with reference to Figure 1B, the axial walls 81 may include a chamfer 86 at their proximal end 85 for easing insertion of the plug 5 inside the inner sleeve 3.

In the embodiment illustrated in Figure 1B, the proximal end portion 7 is defined by the proximal end 85 of the axial walls 81. The proximal end portion 7 has a proximal end surface 71 configured to act as a pushing surface for pushing the needle shield 110 in the proximal direction when the plug 5 is being moved to the assembled position (Figure 1D). Preferably, the proximal end surface 71 is flat and orthogonal to the longitudinal axis A. Since the plug 5 includes four axial walls 81 in the embodiment illustrated in Figure 1B, the proximal end surface 71 is X-shaped.

The pushing force exerted by the plug 5 on the needle shield 110 causes the needle shield 110, together with the medical container 120, to move back in the proximal direction until the proximal end 111 of the needle shield 110 abuts against the axial abutment surface 42 of the hooks 41 or is only separated of the axial abutment surface 42 of the hooks 41 by manufacturing tolerances. As a result, the hooks 41 engage the axial gap 113 between the barrel 123 and the needle shield 110 and the legs 4 return to their rest position. The risks are therefore avoided that a creep effect occurs to the flexible legs 4 over time. Besides, the push-back force is exerted directly on the plug 5, instead of being directly exerted on the needle shield 110.

To that end, the proximal end portion 7 of the plug 5 is axially movable between a first position (Figure 1C) in which the proximal end portion 7 allows the proximal end 111 of the needle shield 110 to go past the the axial abutment surface 42 of the clamping means in the distal direction, and a second position (Figure 1D), proximal to the first position, in which the proximal end portion 7 of the plug 5 abuts against the needle shield 110 and moves the needle shield 110 back in the proximal direction towards or against the axial abutement surface of the clamping means. Here, since all the plug 5 is movable, the first position corresponds to the pre-assembled position of the plug 5 and the second position corresponds to the assembled position of the plug 5. In the second position, the proximal end portion 7 is closer to the axial abutment surface 42 than in the first position.

The operation of the cap 1 is shown in Figures 1A, 1C and 1D.

In Figure 1A, the bottom housing 104 has not yet received the medical container 120. The plug 5 is temporarily maintained in the pre-assembled position by the securing means, i.e. the radial protrusions 84, which engage the lateral wall of the inner cavity 34 at a first predetermined location. The proximal shoulder 62 of the plug 5 is away from the axial shoulder 33 of the distal opening 31.

In Figure 1C, the medical container 120 has been introduced in the lower sub-assembly 102 and pushed in the distal direction until the ring 109 abuts against the rotating cam 107. The needle shield 110 has passed over the hooks 41 of the clamping means, and the legs 4 have accordingly moved to their deflected position. The hooks 41 now rest against the barrel 123 of the medical container 120 and therefore cannot return to their rest position. The plug 5 has not moved yet and is still in the pre-assembled position. The proximal end surface 71 of the plug 5 may also be away from the needle shield 110.

In Figure 1D, a proximal pushing force has been exerted on the distal end surface 61 of the plug 5. As a result, the plug 5 has moved in the proximal direction. The proximal end surface 71 of the plug 5 has come into contact with the needle shield 110 and pushed the needle shield 110 and the medical container 120 back in the proximal direction until the plug 5 is stopped by the axial shoulder 33 of the distal opening 31. As a result of the proximal movement of the needle shield 110 and the medical container 120 due to the pressure of the plug 5, the axial gap 113 between the barrel 123 and the needle shield 110 has moved at the level of the hooks 41, which now engage this axial gap 113. The legs 4 are no longer deflected and have returned to their rest position. The axial abutment surface 42 of the hooks 41 now faces the proximal end 111 of the needle shield 110. Thus, withdrawal of the cap 1 will reliably entail removal of the needle shield 110.

As visible in Figure 1D, the plug 5 is now in the assembled position. The proximal shoulder 62 of the plug 5 abuts against the axial shoulder 33 of the cap 1. The proximal end surface 71 of the plug 5 abuts against the needle shield 110. The plug 5 is maintained in the assembled position by the securing means which engage the lateral wall of the inner cavity 34 at a second predetermined location proximally located relative to the first predetermined location. Due to the abutment between the plug 5 and the needle shield 110, the needle shield 110 and the medical container 120 cannot move in the distal direction during the storage period. The legs 4 remain in their rest position and risks that a creep effect occurs are avoided.

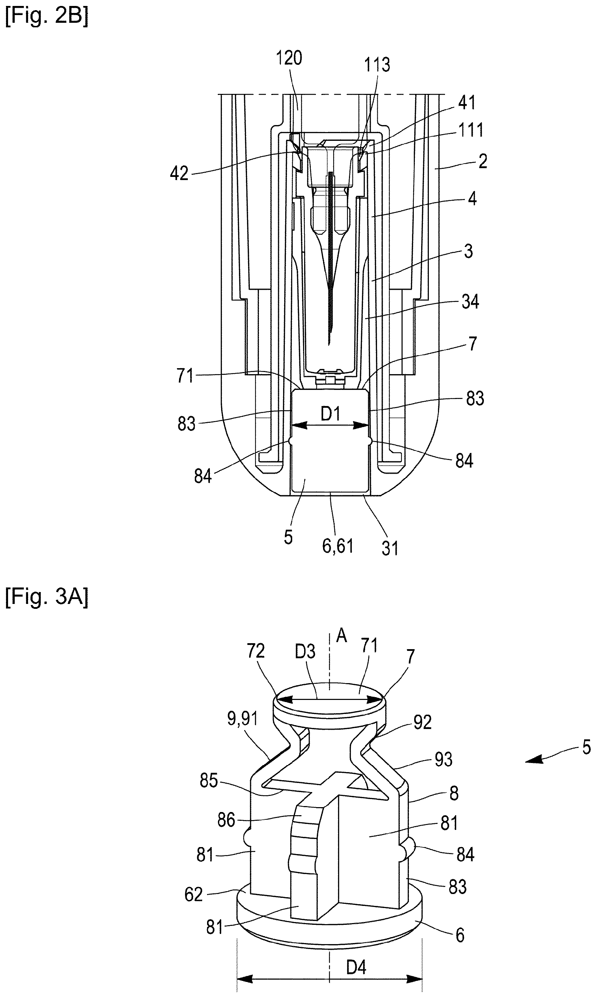

Turning now to Figures 2A and 2B is shown a plug 5 according to another embodiment of the cap 1 of the invention. Similar features with the other embodiments are designated by the same numeral references.

Just as the embodiment of Figures 1A to 1D, the plug 5 of Figure 2A is axially movable with regard to the inner sleeve 3 between a pre-assembled position (not shown) in which the plug 5 partially extends outside the inner cavity 34 and distally protrudes from the distal end 21 of the cap 1, and an assembled position (Figure 2B) in which the plug 5 is fully inserted within the inner sleeve 3.

The plug 5 illustrated in Figure 2A is similar to the plug 5 shown in Figure 1A, except that the proximal end portion 7 is also shaped to fit the inner cavity 34 and seal the distal opening 31. That is, the proximal end portion 7 and the distal end portion 6 of the plug 5 are similar. The plug 5 is symmetrical with regard to a transversal plane orthogonal to the longitudinal axis A. As a result, it is indifferent whether the distal end portion 6 or the proximal end portion 7 of the plug 5 is inserted first. There is no need to turn the plug 5 upside down before assembly. This simplifies the assembly of the cap 1.

More specifically, the distal end portion 6 has no proximal shoulder 62. The diameter of the proximal end portion 7 is the same as the diameter D1 defined by two diametrically opposite axial edge 83 of the axial walls 81. It is also the same as the diameter of the distal end portion 6. The axial edge 83 of the axial walls 81 and the edges of the distal end portion 6 and the proximal end portion 7 accordingly extend at the same distance from the central longitudinal axis A.

Besides, as visible in Figure 2B, the distal opening 31 of the cap 1 has no recess 32 for accommodating the distal end portion 6 of the plug 5 and thus the inner cavity 34 is devoid of an axial shoulder 33. As a result, insertion of the plug 5 inside the inner cavity 34 is stopped by the proximal end 111 of the needle shield 110 abutting against the axial abutment surface 42 of the hooks 41.

It is also contemplated that the proximal end portion 7, and the similar distal end portion 6, has a chamfer 72 extending around the proximal end surface 71, respectively around the . This chamfer 72 eases insertion of the plug 5 inside the inner cavity 34.

In the pre-assembled position, the plug 5 is temporarily maintained by the securing means, i.e. the radial protrusions 84, at a first predetermined location. The proximal end surface 71 of the plug 5 may be away from the needle shield 110.

In the assembled position (Figure 2B), a proximal pushing force has been exerted on the distal end surface 61 of the plug 5. As a result, the plug 5 has moved in the proximal direction. The proximal end surface 71 of the plug 5 has come into contact with the needle shield 110 and pushed the needle shield 110 and the medical container 120 back in the proximal direction until the plug 5 is stopped by the axial abutment surface 42 of the hooks 41 which abuts against the proximal end 111 of the needle shield 110. As a result of the proximal movement of the needle shield 110 and the medical container 120 due to the pressure of the plug 5, the axial gap 113 between the barrel 123 and the needle shield 110 has moved at the level of the hooks 41, which now engage this axial gap 113. The legs 4 are no longer deflected and have returned to their rest position. Thus, withdrawal of the cap 1 will reliably entail removal of the needle shield 110.

To that end, the proximal end portion 7 of the plug 5 is axially movable between a first position (not shown) in which the proximal end portion 7 allows the proximal end of the needle shield 110 to go past the the axial abutment surface 42 of the clamping means in the distal direction, and a second position (Figure 2B), proximal to the first position, in which the proximal end portion 7 of the plug 5 abuts against the needle shield 110 and moves the needle shield 110 back in the proximal direction towards or against the axial abutement surface of the clamping means. Here, since all the plug 5 is movable, the first position corresponds to the pre-assembled position of the plug 5 and the second position corresponds to the assembled position of the plug 5. In the second position, the proximal end portion 7 is closer to the axial abutment surface 42 than in the first position.

As visible in Figure 2B, the proximal end surface 71 of the plug 5 abuts against the needle shield 110. The plug 5 is maintained in the assembled position by the securing means which engage the lateral wall of the inner cavity 34 at a second predetermined location proximally located relative to the first predetermined location. Due to the abutment between the plug 5 and the needle shield 110, the needle shield 110 and the medical container 120 cannot move in the distal direction during the storage period. The legs 4 remain in their rest position and risks that a creep effect occurs are avoided.

Unlike the embodiment of Figure 1D, the distal end surface 61 of the plug 5 in the assembled position may however not be flush with the distal end 21 of the cap 1. Instead, this distal end surface 61 of the plug 5 may be located slightly proximal to said distal end 21 of the cap 1.

Turning now to Figures 3A-3C is shown a plug 5 according to another embodiment of the cap 1 of the invention. Similar features with the other embodiments are designated by the same numeral references.

The plug 5 of Figures 3A-3C is similar to the plug 5 shown in Figures 1A-1D, except that the plug 5 of Figures 3A-3C further includes a spring portion 9. The spring portion 9 is configured to resiliently bias the proximal end portion 7 of the plug 5 in the proximal direction. The spring portion 9 may be arranged between the intermediate portion 8 and the proximal end portion 7 of the plug 5.

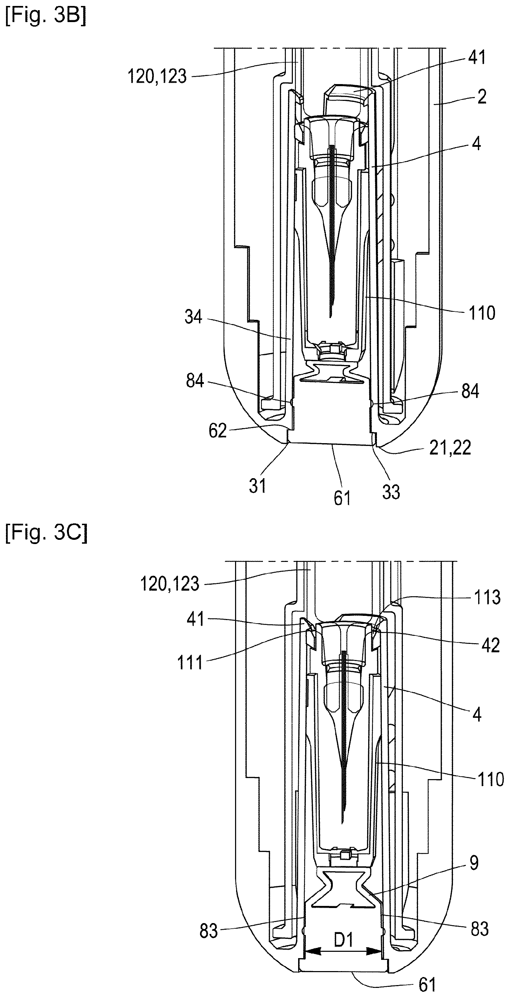

With reference to Figure 3A, the spring portion 9 includes two resiliently deformable arms 91, movable between a rest position (Figure 3A), in which the spring portion 9 maintains the proximal end portion 7 at a stable axial position, and a deformed position (Figure 3B), in which the spring portion 9 is compressed and in which the proximal end portion 7 of the plug 5 has moved distally relative to the stable axial position. The spring portion 9 may be V-shaped. The arms 91 may include an upper and a lower inclined branches joining each other towards the central longitudinal axis A. The upper branch 92 may be shorter than the lower branch 93. The upper branch 92 connects the lower branch 93 to the proximal end portion 7 (and more specifically to a distal surface of the proximal end portion 7), while the lower branch 93 connects the upper branch 92 to the intermediate portion 8 (and more specifically to a proximal end 85 of the axial walls 81).

As visible in Figure 3A, the proximal end portion 7 of the plug 5 is not formed by the proximal end surface 71 of the axial walls 81, but is instead separated from the axial walls 81 by the spring portion 9. This proximal end portion 7 may be disc-shaped to better distribute the effort exerted by the spring against the needle shield 110. The proximal end portion 7 preferably has a diameter D3 which is lower than a diameter D4 of the distal end portion 6 and the inner diameter D2 of the inner sleeve 3. The diameter D3 may also be lower than the diameter D1 of the axial edges 83. Thus, the proximal end portion 7 of the plug 5 does not hamper insertion of the plug 5 inside the inner cavity 34. The proximal end portion 7 may also include a chamfer 72 surrounding the proximal end surface 71 in order to ease insertion of the plug 5 inside the inner cavity 34.

The proximal end portion 7 of the plug 5 is axially movable between a first position (Figure 3B) in which the proximal end portion 7 allows the proximal end 111 of the needle shield 110 to go past the the axial abutment surface 42 of the clamping means in the distal direction, and a second position (Figure 3C), proximal to the first position, in which the proximal end portion 7 of the plug 5 abuts against the needle shield 110 and moves the needle shield 110 back in the proximal direction towards or against the axial abutement surface of the clamping means. Here, since the plug 5 may be fixed, the first position corresponds to the deformed position of the spring portion 9 of the plug 5 and the second position corresponds to the rest position or a less deformed position of the spring portion 9 of the plug 5. In the second position, the proximal end portion 7 is closer to the axial abutment surface 42 than in the first position.

The operation of the cap 1 is shown in Figures 3B and 3C.

Before insertion of the medical container 120 into the bottom housing 104, it is contemplated that the plug 5 may either be in a pre-assembled position (similar to the one illustrated in Figure 1C) or may already be in an assembled position in which the plug 5 is fully inserted inserted inside the cap 1.

In Figure 3B, the medical container 120 has been introduced in the bottom housing 104 and pushed in the distal direction until the ring 109 abuts against the rotating cam 107. The needle shield 110 has passed over the hooks 41 of the clamping means, and the legs 4 have accordingly moved to their deflected position. The hooks 41 now rest against the barrel 123 of the medical container 120 and therefore cannot return to their rest position.

Still with reference to Figure 3B, the plug 5 is illustrated in the assembled position. The proximal shoulder 62 of the plug 5 abuts against the axial shoulder 33 of the distal opening 31. The plug 5 does not distally protrude from the distal end 21 of the cap 1. The distal surface of the plug 5 is flush with the circumferential flat surface 22 of the cap 1. The securing means, i.e. the radial protrusions 84, engage the inner lateral wall of the inner cavity 34 and thus maintain the plug 5 in the assembled position. Besides, the proximal end portion 7 of the plug 5 abuts against the needle shield 110 and the spring portion 9 is in the deformed position. As a result, the spring portion 9 the plug 5 exerts a proximal pushing force on the needle shield 110.

In Figure 3C, due to the pressure of the spring portion 9, the needle shield 110 and the medical container 120 have moved back in the proximal direction until the proximal end 111 of the needle shield 110 abuts against the hooks 41. As a result of the proximal movement of the needle shield 110 and the medical container 120 due to the pressure of the spring portion 9, the axial gap 113 between the barrel 123 and the needle shield 110 has moved at the level of the hooks 41, which now engage this axial gap 113. The legs 4 are no longer deflected and have returned to their rest position. The axial abutment surface 42 of the hooks 41 now abuts against the proximal end 111 of the needle shield 110. Thus, withdrawal of the cap 1 will reliably entail removal of the needle shield 110.

In Figure 3C, due to the abutment between the plug 5 and the needle shield 110 and the effort of the spring portion 9, the needle shield 110 and the medical container 120 cannot move in the distal direction during the storage period. The legs 4 remain in their rest position and risks that a creep effect occurs are avoided.

The embodiment of Figures 3A-3C allows that the push-back force is exerted by the plug 5 itself, instead of being exerted by an external machine or operator, so that there is no need for an additional push-back step during the assembly process. That is, the push-back step is automatically realized by the plug 5 itself.

As illustrated in Figures 1A, 2B and 3B, the cap 1 of the invention is preferably a two-part cap 1. That is, the plug 5 and the tubular body 2 of the cap 1 are preferably two distinct components. With reference to Figures 1B, 2A and 3A, the plug 5 may be made of a single piece, for example by injection molding. The material of the plug 5 may include acrylonitrile-butadienestyrene (ABS), polycarbonate (PC), polyoxymethylene (POM) and any combinations thereof. The plug 5 may also include any other appropriate material. In an alternative embodiment not shown, the plug 5 of Figures 3A-3C might however be integral with the inner sleeve 3 and the tubular body 2

Preferably, the plug 5 is rotationally symmetrical around the longitudinal axis A (for instance a 90° or 180° rotation in the embodiments illustrated in Figures 1B, 2A and 3A). Accordingly, insertion of the plug 5 into the inner cavity 34 is easily done whatever the angular orientation of the plug 5 with regard to the longitudinal axis A and the inner sleeve 3.

The invention also relates to a method for assembling a medical container 120 having a needle shield 110 with an autoinjector 100 including the above-described cap 1. The method includes a step of inserting the medical container 120 inside the bottom housing 104. During this step, the needle shield 110 deflects the flexible legs 4 and enters the inner sleeve 3. Insertion of the needle shield 110 inside the inner cavity 34 is stopped by the ring 109 abutting against the cam 107 of the lower sub-assembly 102. At this stage, the needle shield 110 has passed over the hooks 41 and the proximal end 111 of the needle shield 110 is distally distant from the axial abutment surface 42 of the hooks 41. Since the hooks 41 bear against the barrel 123 of the medical container 120, and thus are in a deflected position, it is now required to push the needle shield 110 and the medical container 120 back in the proximal direction. Thus, the method includes a step of pushing the needle shield 110 back in the proximal direction by means of the plug 5. Either the plug 5 automatically pushes the needle shield 110 back by means of the spring portion 9 (Figures 3A-3C), or a machine or an operator moves the plug 5 from the pre-assembled to the assembled position (Figures 1C-1D and 2B). Movement of the plug 5 from the pre-assembled to the assembled position causes the plug 5 to abut against the needle shield 110 and push the needle shield 110 in the distal direction. Either the movement of the plug 5 is stopped by its proximal shoulder 62 abutting against the axial shoulder 33 of the cap 1 (Figure 1D), i.e. the assembly is "stroke driven", or the movement of the plug 5 is stopped by the needle shield 110 abutting against the hooks 41 (Figure 2B), i.e. the assembly is "force driven". In the first case (Figure 1D), a small distance may still separate the hooks 41 and the needle shield 110, due to the manufacturing tolerances. In the second case (Figure 2B), the needle shield 110 and the hooks 41 abut against each other. In all cases however, the hooks 41 have returned to their rest position and engage the room between the barrel 123 and the needle shield 110, so that the axial abutment surface 42 of the hooks 41 necessarily engage the proximal end 111 of the needle shield 110 when the cap 1 is withdrawn. This ensures removal of the needle shield 110.

It is to be understood that the present invention is not limited to the embodiments described above and illustrated in the drawings; rather, the skilled person will recognize that many changes and modifications may be made within the scope of the appended claims.

## Claims

1. A cap (1) for removing a needle shield (110) attached to a medical container (120) provided with an injection needle (121), the cap (1) including:
a tubular body (2) extending along a longitudinal axis A, the tubular body (2) having a distal end (21) and an opposite proximal end (20) for attachment to a bottom housing (104) of an autoinjector (100),
an inner sleeve (3) proximally extending inside the tubular body (2) from said distal end (21), the inner sleeve (3) defining an inner cavity (34) for receiving the needle shield (110),
the inner sleeve (3) having clamping means for clamping the needle shield (110) when the needle shield (110) is inserted inside the inner cavity (34), the clamping means including an axial abutment surface (42) for abutting against a proximal end (111) of the needle shield (110),
a plug (5) at least partially extending inside the inner cavity (34), the plug (5) having a distal end portion (6), a proximal end portion (7) and an intermediate portion (8) extending between the distal end portion (6) and the proximal end portion (7),
wherein at least the proximal end portion (7) of the plug (5) is movable between a first position, in which the proximal end portion (7) allows the proximal end (111) of the needle shield (110) to distally pass the axial abutment surface (42) of the clamping means, and a second position, proximal to the first position, in which the proximal end portion (7) of the plug (5) moves in the proximal direction to push the needle shield (110) back towards the axial abutment surface (42).

2. The cap (1) according to the preceding claim, wherein the distal end portion (6) of the plug (5) and a distal opening (31) of the inner cavity (34) have a complementary shape.

3. The cap (1) according to any of the preceding claims, wherein the intermediate portion (8) of the plug (5) includes one or more axial walls (81) for centering and securing the plug (5) inside the inner cavity (34).

4. The cap (1) according to any of the preceding claims, wherein the proximal end portion (7) of the plug (5) includes a chamfer (72).

5. The cap (1) according to any of the preceding claims, wherein the distal end portion (6) of the plug (5) is flush with regard to the distal end (21) of the tubular body (2) in the second position.

6. The cap (1) according to any of the preceding claims, wherein the distal end portion (6) of the plug (5) has a proximal shoulder (62) for abutting against an axial shoulder (33) of the inner cavity (34) in the second position.

7. The cap (1) according to any of the preceding claims, wherein a diameter (D3) of the proximal end portion (7) is lower than a diameter (D4) of the distal end portion (6).

8. The cap (1) according to any of claims 1-5, wherein the proximal end portion (7) and the distal end portion (6) of the plug (5) are symmetrical with regard to a transversal plane orthogonal to the longitudinal axis A.

9. The cap (1) according to any of the preceding claims 1-7, wherein the intermediate portion (8) of the plug (5) includes a spring portion (9) for biasing the proximal end portion (7) in the proximal direction.

10. The cap (1) according to any of the preceding claims, wherein the proximal end portion (7) of the plug (5) is disc-shaped.

11. The cap (1) according to claim 3 or any of claims 4-7 when depending on claim 3, wherein the proximal end portion (7) of the plug (5) is formed by a proximal end (85) of said one or more axial walls (81).

12. The cap (1) according to any of the preceding claims, wherein the plug (5) is made of a single piece.

13. The cap (1) according to any of the preceding claims, wherein the plug (5) is rotationally symmetrical around the longitudinal axis A.

14. An autoinjector (100) including a lower sub-assembly (102) provided with the cap (1) of any of the preceding claims.

15. A method for assembling the autoinjector (100) of claim 14 and a medical container (120) having a needle shield (110), the method including the steps of:
(i) inserting the medical container (120) inside the lower sub-assembly (102) of the autoinjector (100) and the needle shield (110) inside the inner cavity (34) of the cap (1) until a proximal end (111) of the needle shield (110) distally passes over the axial abutment surface (42) of the clamping means;
(ii) pushing the needle shield (110) back in the proximal direction towards the axial abutment surface (42) of the clamping means, this movement of the needle shield (110) being caused by the proximal end portion (7) of the plug (5) moving from the first position to the second position.

## Patentansprüche

1. Kappe (1) zum Entfernen eines Nadelschutzes (110), der an einem medizinischen Behälter (120) angebracht ist, der mit einer Injektionsnadel (121) versehen ist, wobei die Kappe (1) Folgendes einschließt:
einen sich entlang einer Längsachse A erstreckenden Rohrkörper (2), wobei der Rohrkörper (2) ein distales Ende (21) und ein gegenüberliegendes proximales Ende (20) zur Befestigung an einem unteren Gehäuse (104) eines Autoinjektors (100) aufweist,
eine innere Hülse (3), die sich proximal innerhalb des Rohrkörpers (2) von dem distalen Ende (21) erstreckt, wobei die innere Hülse (3) einen inneren Hohlraum (34) zur Aufnahme des Nadelschutzes (110) definiert,
wobei die Innenhülse (3) Klemmmittel zum Klemmen des Nadelschutzes (110) aufweist, wenn der Nadelschutz (110) in den inneren Hohlraum (34) eingeführt wird, wobei die Klemmmittel eine axiale Anschlagfläche (42) zum Anliegen an einem proximalen Ende (111) des Nadelschutzes (110) einschließen,
einen Stopfen (5), der sich mindestens teilweise innerhalb des inneren Hohlraums (34) erstreckt, wobei der Stopfen (5) einen distalen Endabschnitt (6), einen proximalen Endabschnitt (7) und einen zwischen dem distalen Endabschnitt (6) und dem proximalen Endabschnitt (7) erstreckenden Zwischenabschnitt (8) aufweist,
wobei mindestens der proximale Endabschnitt (7) des Stopfens (5) zwischen einer ersten Position, in der der proximale Endabschnitt (7) es dem proximalen Ende (111) des Nadelschutzes (110) ermöglicht, distal über die axiale Anschlagfläche (42) der Klemmmittel zu verlaufen, und einer zweiten Position, proximal der ersten Position, in der sich der proximale Endabschnitt (7) des Stopfens (5) in proximaler Richtung bewegt, um den Nadelschutz (110) zurück in Richtung der axialen Anschlagfläche (42) zu drücken, verschiebbar ist.

2. Kappe (1) nach dem vorhergehenden Anspruch, wobei der distale Endabschnitt (6) des Stopfens (5) und eine distale Öffnung (31) des inneren Hohlraums (34) eine komplementäre Form aufweisen.

3. Kappe (1) nach einem der vorhergehenden Ansprüche, wobei der Zwischenabschnitt (8) des Stopfens (5) eine oder mehrere axiale Wände (81) zum Zentrieren und Sichern des Stopfens (5) innerhalb des Innenhohlraums (34) einschließt.

4. Kappe (1) nach einem der vorhergehenden Ansprüche, wobei der proximale Endabschnitt (7) des Stopfens (5) eine Fase (72) einschließt.

5. Kappe (1) nach einem der vorhergehenden Ansprüche, wobei der distale Endabschnitt (6) des Stopfens (5) in Bezug auf das distale Ende (21) des Rohrkörpers (2) in der zweiten Position bündig ist.

6. Kappe (1) nach einem der vorhergehenden Ansprüche, wobei der distale Endabschnitt (6) des Stopfens (5) eine proximale Schulter (62) zum Anliegen an einer axialen Schulter (33) des Innenhohlraums (34) in der zweiten Position aufweist.

7. Kappe (1) nach einem der vorhergehenden Ansprüche, wobei ein Durchmesser (D3) des proximalen Endabschnitts (7) kleiner ist als ein Durchmesser (D4) des distalen Endabschnitts (6).

8. Kappe (1) nach einem der Ansprüche 1 bis 5, wobei der proximale Endabschnitt (7) und der distale Endabschnitt (6) des Stopfens (5) in Bezug auf eine Querebene orthogonal zu der Längsachse A symmetrisch sind.

9. Kappe (1) nach einem der vorhergehenden Ansprüche 1 bis 7, wobei der Zwischenabschnitt (8) des Stopfens (5) einen Federabschnitt (9) zum Biegen des proximalen Endabschnitts (7) in proximaler Richtung einschließt.

10. Kappe (1) nach einem der vorhergehenden Ansprüche, wobei der proximale Endabschnitt (7) des Stopfens (5) scheibenförmig ist.

11. Kappe (1) nach Anspruch 3 oder einem der Ansprüche 4 bis 7, wenn abhängig von Anspruch 3, wobei der proximale Endabschnitt (7) des Stopfens (5) durch ein proximales Ende (85) der einen oder mehreren axialen Wände (81) gebildet ist.

12. Kappe (1) nach einem der vorhergehenden Ansprüche, wobei der Stopfen (5) aus einem Stück besteht.

13. Kappe (1) nach einem der vorhergehenden Ansprüche, wobei der Stopfen (5) um die Längsachse A rotationssymmetrisch ist.

14. Autoinjektor (100), der eine untere Unterbaugruppe (102) mit der Kappe (1) nach einem der vorhergehenden Ansprüche einschließt.

15. Verfahren zum Zusammenbauen des Autoinjektors (100) nach Anspruch 14 und eines medizinischen Behälters (120), der einen Nadelschutz (110) aufweist, wobei das Verfahren folgende Schritte einschließt:
(i) Einsetzen des medizinischen Behälters (120) in die untere Unterbaugruppe (102) des Autoinjektors (100) und des Nadelschutzes (110) in den inneren Hohlraum (34) der Kappe (1), bis ein proximales Ende (111) des Nadelschutzes (110) distal über die axiale Anschlagfläche (42) der Klemmmittel verläuft;
(ii) Zurückschieben des Nadelschutzes (110) in proximaler Richtung zu der axialen Anschlagfläche (42) der Klemmmittel, wobei diese Bewegung des Nadelschutzes (110) dadurch verursacht wird, dass sich der proximale Endabschnitt (7) des Stopfens (5) von der ersten Position in die zweite Position bewegt.

## Revendications

1. Capuchon (1) pour retirer un protecteur d'aiguille (110) fixé à un récipient médical (120) muni d'une aiguille d'injection (121), le capuchon (1) comprenant :
un corps tubulaire (2) s'étendant le long d'un axe longitudinal A, le corps tubulaire (2) ayant une extrémité distale (21) et une extrémité proximale opposée (20) pour la fixation à un boîtier inférieur (104) d'un auto-injecteur (100),
un manchon interne (3) s'étendant de manière proximale à l'intérieur du corps tubulaire (2) depuis ladite extrémité distale (21), le manchon interne (3) définissant une cavité interne (34) pour recevoir le protecteur d'aiguille (110),
le manchon interne (3) ayant des moyens de serrage pour serrer le protecteur d'aiguille (110) lorsque le protecteur d'aiguille (110) est inséré à l'intérieur de la cavité interne (34), les moyens de serrage comprenant une surface de butée axiale (42) pour venir en butée contre une extrémité proximale (111) du protecteur d'aiguille (110),
un bouchon (5) s'étendant au moins partiellement à l'intérieur de la cavité interne (34), le bouchon (5) ayant une partie d'extrémité distale (6), une partie d'extrémité proximale (7) et une partie intermédiaire (8) s'étendant entre la partie d'extrémité distale (6) et la partie d'extrémité proximale (7),
dans lequel au moins la partie d'extrémité proximale (7) du bouchon (5) est mobile entre une première position, dans laquelle la partie d'extrémité proximale (7) permet à l'extrémité proximale (111) du protecteur d'aiguille (110) de passer distalement la surface de butée axiale (42) des moyens de serrage, et une seconde position, proximale par rapport à la première position, dans laquelle la partie d'extrémité proximale (7) du bouchon (5) se déplace dans la direction proximale pour repousser le protecteur d'aiguille (110) vers la surface de butée axiale (42).

2. Capuchon (1) selon la revendication précédente, dans lequel la partie d'extrémité distale (6) du bouchon (5) et une ouverture distale (31) de la cavité interne (34) ont une forme complémentaire.

3. Capuchon (1) selon l'une des revendications précédentes, dans lequel la partie intermédiaire (8) du bouchon (5) comprend une ou plusieurs parois axiales (81) pour centrer et fixer le bouchon (5) à l'intérieur de la cavité interne (34).

4. Capuchon (1) selon l'une des revendications précédentes, dans lequel la partie d'extrémité proximale (7) du bouchon (5) comprend un chanfrein (72).

5. Capuchon (1) selon l'une des revendications précédentes, dans lequel la partie d'extrémité distale (6) du bouchon (5) affleure l'extrémité distale (21) du corps tubulaire (2) dans la seconde position.

6. Capuchon (1) selon l'une des revendications précédentes, dans lequel la partie d'extrémité distale (6) du bouchon (5) a un épaulement proximal (62) pour venir en butée contre un épaulement axial (33) de la cavité interne (34) dans la seconde position.

7. Capuchon (1) selon l'une des revendications précédentes, dans lequel un diamètre (D3) de la partie d'extrémité proximale (7) est inférieur à un diamètre (D4) de la partie d'extrémité distale (6).

8. Capuchon (1) selon l'une des revendications 1 à 5, dans lequel la partie d'extrémité proximale (7) et la partie d'extrémité distale (6) du bouchon (5) sont symétriques par rapport à un plan transversal orthogonal à l'axe longitudinal A.

9. Capuchon (1) selon l'une des revendications précédentes 1 à 7, dans lequel la partie intermédiaire (8) du bouchon (5) comprend une partie ressort (9) pour solliciter la partie d'extrémité proximale (7) dans la direction proximale.

10. Capuchon (1) selon l'une des revendications précédentes, dans lequel la partie d'extrémité proximale (7) du bouchon (5) est en forme de disque.

11. Capuchon (1) selon la revendication 3 ou l'une des revendications 4 à 7 lorsqu'elles dépendent de la revendication 3, dans lequel la partie d'extrémité proximale (7) du bouchon (5) est formée par une extrémité proximale (85) de ladite ou desdites plusieurs parois axiales (81).

12. Capuchon (1) selon l'une des revendications précédentes, dans lequel le bouchon (5) est réalisé en une seule pièce.

13. Capuchon (1) selon l'une des revendications précédentes, dans lequel le bouchon (5) est symétrique en rotation autour de l'axe longitudinal A.

14. Auto-injecteur (100) comprenant un sous-ensemble inférieur (102) muni du capuchon (1) selon l'une des revendications précédentes.

15. Procédé d'assemblage de l'auto-injecteur (100) selon la revendication 14 et d'un récipient médical (120) ayant un protecteur d'aiguille (110), le procédé comprenant les étapes consistant à :
(i) insérer le récipient médical (120) à l'intérieur du sous-ensemble inférieur (102) de l'auto-injecteur (100) et le protecteur d'aiguille (110) à l'intérieur de la cavité interne (34) du capuchon (1) jusqu'à ce qu'une extrémité proximale (111) du protecteur d'aiguille (110) passe distalement par-delà la surface de butée axiale (42) des moyens de serrage ;
(ii) repousser le protecteur d'aiguille (110) dans la direction proximale vers la surface de butée axiale (42) des moyens de serrage, ce mouvement du protecteur d'aiguille (110) étant provoqué par le déplacement de la partie d'extrémité proximale (7) du bouchon (5) de la première position à la seconde position.
